# EUROPEAN PATENT SPECIFICATION

(11) **EP 2 170 954 B1**
(45) Date of publication and mention of the grant of the patent: **14.08.2013**
(21) Application number: 08779117.4
(22) Date of filing: 12.06.2008
(51) Int. Cl.: G01N 33/53, G01N 33/50, C07K 16/18, A61K 39/00, A61P 33/00

(54) **METHODS OF SELECTING HOST RESISTANT ANIMALS**
VERFAHREN ZUR AUSWAHL VON RESISTENTEN WIRTSTIEREN
PROCÉDÉS DE SÉLECTION D'ANIMAUX RÉSISTANTS HÔTES

(30) Priority: 13.06.2007 NZ 55584707; 06.05.2008 NZ 56801708
(43) Date of publication of application: 07.04.2010
(73) Proprietor: Ovita Limited, Dunedin (NZ)
(72) Inventor: SHAW, Richard, John, Palmerston North (NZ); CANNON, Merie, Christine, Otaki (NZ); ROSANOWSKI, Sarah, Margaret, Fielding (NZ); WHEELER, Mary, Hamilton (NZ); MORRIS, Christopher, Anthony, Hamilton (NZ)
(74) Representative: Brady, Paul Andrew
(86) International application number: PCT/NZ2008/000139
(87) International publication number: WO 2008/153420

(56) References cited:
- WO-A1-03/064475
- WO-A1-03/064475
- MARTINEZ-VALLADARES ET AL.: "Genetic resistance to Teladorsagia circumcinta: IgA and parameters at Slaughter in Churra sheep" PARASITE IMMUNOLOGY, vol. 27, 2005, pages 213-218, XP002587990
- DOUCH P.G. ET AL.: "Phenotype markers for selection of nematode-resistant sheep" INTERNATIONAL JOURNAL FOR PARASITOLOGY, vol. 26, no. 8-9, 1996, pages 899-911, XP002587991
- HARRISON G B L ET AL: "Immune rejection of Trichostrongylus colubriformis in sheep; a possible role for intestinal mucus antibody against an L3-specific surface antigen." PARASITE IMMUNOLOGY JAN 2003 LNKD- PUBMED:12753437, vol. 25, no. 1, January 2003 (2003-01), pages 45-53, XP002587992 ISSN: 0141-9838 & HARRISON G B L ET AL: "Characterization of a 35-kDa carbohydrate larval antigen (CarLA) from Trichostrongylus colubriformis; a potential target for host immunity." PARASITE IMMUNOLOGY (OXFORD), vol. 25, no. 2, February 2003 (2003-02), pages 79-86, XP2350701 ISSN: 0141-9838
- SHAW R J ET AL: "Genetic and phenotypic relationships among Trichostrongylus colubriformis-specific immunoglobulin E, anti-Trichostrongylus colubriformis antibody, immunoglobulin G1, faecal egg count and body weight traits in grazing Romney lambs" LIVESTOCK PRODUCTION SCIENCE, vol. 58, no. 1, March 1999 (1999-03), pages 25-32, XP002587993 ISSN: 0301-6226
- MORRIS C A ET AL: "Continued selection of Romney sheep for resistance or susceptibility to nematode infection: Estimates of direct and correlated responses" ANIMAL SCIENCE (PENCAITLAND), vol. 70, no. 1, February 2000 (2000-02), pages 17-27, XP002587994 ISSN: 1357-7298
- MARTINEZ-VALLADARES ET AL.: 'Genetic resistance to Teladorsagia circumcinta: IgA and parameters at Slaughter in Churra sheep' PARASITE IMMUNOLOGY vol. 27, 2005, pages 213 - 218, XP002587990
- HARRISON G.B.I. ET AL.: 'Studies on the role of mucus and mucosal hypersensitivity reactions during rejection of Trichostrongylus colubriformis from the intestine of immune sheep using an experimental challenge model' INTERNATIONAL JOURNAL FOR PARASITOLOGY vol. 29, 1999, pages 459 - 468, XP008128054
- JACOBS H.J. ET AL.: 'Vaccination against the gastrointestinal nematode, Haemonchus/contortus using a purified larval surface antigen' VACCINE vol. 17, 1999, pages 62 - 68, XP004144748
- DOUCH P.G. ET AL.: 'Phenotype markers for selection of nematode-resistant sheep' INTERNATIONAL JOURNAL FOR PARASITOLOGY vol. 26, no. 8-9, 1996, pages 899 - 911, XP002587991
- NEEDHAM C.S. ET AL.: 'Potential for diagnosis of intestinal nematode infections through antibody detection in saliva' TRANSACTION OF THE ROYAL SOCIETY OF TROPICAL MEDICINE AND HYGIENE no. 90, 1996, pages 526 - 530, XP008128055

## Description

### Field of the Invention

The present invention relates to a method of selecting animals that are parasite resistant, and to a diagnostic test for detecting parasite resistance in animals. In particular, the diagnostic test is based on the detection of antibodies to parasite antigens in mucosal fluid from the animal. Specifically, although by no means exclusively, the invention relates to selecting sheep and cattle that are resistant to nematode worm infection.

No statement in this specification should be construed as relating to an essential biological process, in particular as a process involving a step of crossing and selecting animals.

### Background of the invention

Internal parasites, and especially nematode worm infection, is one of the biggest health problems in sheep and cattle farms around the world. Such internal parasites cause costly reductions in animal performance and, if left untreated, death of the animal.

Traditionally, parasite infections are treated with anthelmintic agents. There are three families of chemicals from which all anthelmintic agents are formulated, including (1) the benzimidazoles; (2) levamisole and morantel; and (3) ivermectin. The effectiveness of these compounds has been reduced as resistance develops in the parasites. Indeed, recent industry-funded surveys in New Zealand have found that 64% of sheep farms and 94% of beef farms now have parasites that are resistant to at least one of the three classes of anthelmintics. Such resistance is estimated to cost the New Zealand sheep industry more than twenty million dollars annually in lost productivity (principally through undetected resistance) and increased expenses (through farmers being forced to use expensive drench products).

There has been no new class of anthelmintic for some time, and it is unlikely that a new class will be available in the foreseeable future that is cost-effective for treating nematode infections unless resistance to all three anthelmintic families has occurred.

Alternative methods of controlling the effect of on-farm parasite infections have been proposed, and include altered grazing management, use of nematode trapping fungi, dietary supplementation, vaccines, and the selective breeding of animals for Host Resistance.

Selecting sheep and cattle that are naturally resistant to intestinal nematodes has been successful in a number of breeds in New Zealand, Australia and Ireland. Such Selecting is currently achieved using faecal egg count (FEC). This method is costly and labour intensive, and requires exposing animals to a reasonable parasite challenge for around six to eight weeks post-weaning. The average of two egg counts taken over this six to eight week period is used to assess the parasite resistance of each animal. It has also been estimated that at least eight years of selection is required before consistently low FECs are obtained.

Other methods for selecting animals that are Host Resistant include methods of selecting animals that show high levels of natural immunity to the parasites. Some sheep studies have shown that this immunity is correlated with reduced FEC and may be effected via antibodies (IgGI, IgE, IgA), eosinophilia, mast cells, goblet cells, fluid and electrolyte secretion, increased smooth muscle activity and increased epithelial cell turnover. However, not all of these immune responses have been shown to be effective at preventing the establishment of larvae or expelling adult nematodes.

WO 03/064475 discloses a possible marker of immune resistance that may be used to select animals with increased levels of genetic resistance. In particular, naturally resistant sheep were shown to produce antibodies to an antigen present in the third stage larvae of parasite nematodes. This antigen consists of a carbohydrate protective coat (CarLA). Antibodies to CarLA have been detected in the intestinal mucosa. The level of antibody in intestinal mucosa has been shown to be correlated to reduced FEC and to prevent larval establishment (Harrison *et al,* 2003). However, mucosal sampling to test animals for Host Resistance is clearly not practical.

WO 03/064475 discloses a diagnostic test that measures the presence of an antibody to CarLA or the CarLA antigen perse in a blood sample. However, such a test was not exemplified in this specification so that it is not clear if antibodies to CarLA or the CarLA antigen are detectable in blood, or, even if they are detectable, whether or not such antibody or antigen levels in blood correlate with Host Resistance.

In addition, even if the blood test described in WO 03/064475 was useful to select resistant animals, such a blood test would be labour intensive and costly as the samples would require testing by professional laboratories. In addition, farmers would be reluctant to use such an invasive test.

A simpler test would be desirable that required easy sample collection, so would be less labour intensive, and less expensive, provide quicker results and was practical for on-farm use.

It is an object of the present invention to go some way towards achieving this desideratum and/or to provide the public with a useful choice.

### Summary of the Invention

In its broadest sense, the invention concerns subject-matter as defined in the appended claims.
1. A method of selecting ungulates that are genetically resistant to one or more nematode worm Infections, said method comprising the steps: a of mucus from
   (a) obtaining a sample of mucus from said ungulate;
   (b) testing the sample for the presence of an antibody against the carbohydrate protective coat antingen (CarlA) present on the third lavral stage (L3) of said one ore more nematode worms; and
   (c) segregating and selecting ungulates that test positive for the antibody In step (b) wherein the mucus sample comprises mucosal fluid from the nose, mouth, throat, rectal cavity or vagina.
2. A method as claimed in claim 1, wherein the mucus sample comprises saliva.
3. A method as claimed in claim 1, wherein the one or more nematode worms are selected from the group consisting of *Trichostrongylus colubriformls, Haemonchus contortus, Ostertagia (Teledorsagia) circumcincta, Cooperia curticel, Nematodirus spathiger, Trichostrongylus axei, Trichostrongylus vitrinus, Ostertagia ostertagi, Cooperia oncophera, Nematodirus brasiliensis* and *Dictyocaulus eckerti.*
4. A method as claimed in any one of claims 1-3, wherein the antibody comprises monoclonal antibody mAb PAB-1, (ATCC accession no. PTA-4005). which specifically binds to the CarLA surface antigen on nematode L3.
5. A method for detecting the presence of antibodies that are specific for an L3 CarLA surface antigen present on one or more species of nematode worms in a mucus sample from an ungulate, said method comprising the steps:
   (a) obtaining a sample of mucus from said ungulate comprising mucosal fluid from the nose, mouth, throat, rectal cavity or vagina.
   (b) contacting said sample with the L3 CarLA surface antigen. thereby forming an antibody/antigen complex; and
   (c) detecting the presence or absence of the complex:
      wherein the presence of said antibody/antigen complex is indicative of an ungulate that is genetically resistant to nematode worm infection.
6. A melhod as claimed in claim 5, wherein the mucus sample is saliva.
7. A method as claimed in claim 5 or 6, wherein the antigen is selected from one or more nematode L3 CarLA antigens selected from the group consisting of;
   (a) a surface antigen on *C.curticei* which runs at substantially 46 kDa and at substantially 22kDa on SDS PAGE gel under reducing conditions;
   (b) a surface antigen on *N.spathiger* which runs at substantially 22kDa on SDS PAGE gel under reducing conditions:
   (c) a surface antigen on *H.contortus* which runs al substantially 35kDa on SDS PAGE' gel under reducing conditions: and
   (d) a surface antigen on *O.circumcincta* which runs at substantially 38-39kDa on SDS PAGE gel under reducing conditions;
   (e) a surface antigen on *T.axel* or *T.vitrinus* which runs at substantially 35kDa on SDS PAGE gel under reducing conditions;
   (f) a surface antigen on *O.ostertegl* which runs at substantially. 30-45kDa on SDS PAGE gel under reducing conditions:
   (g) a surface antigen on *C.oncophere* which runs at subetantially 20 kDa and at substantially 48 kDa on SDS PAGE gel under reducing conditions;
   (h) a surface antigen on *N:bresitiensls* which runs at substantially 9 kDa and at substantially 12 kDa on SDS PAGE gel under reducing conditions; and
   (i) a surface antigen on *D. eckertl* which runs at substantially 30 kDa on SDS PAGE gel under reducing conditions.
8. A method aa claimed in claim 7, wherein the antigen is immobilsed on a solid surface and the complex detected by using a label on the antigen which is detectable when the complex is formed.
9. A method as claimed in claim 8, wherein the complex is detected by using a second labelled antibody which binds to the complex.
10. A method as claimed in claim 8 or 9, wherein the detection is carried out using a teat strip comprising one or more immobilised antigens.
11. A kit for detecting the presence of an antibody specific for one or more nematode L3 CarLA surface antigens, said kit comprising:
   (a) a container for mucus sample;
   (b) a reagent comprising one or more labelled nematode CarLA L3 antigens; and
   (c) a mucus sample collection means;
      wherein the mucus sample is selected from a sample from the nose, mouth, troat, rectal cavity or vagina.
12. A kit as claimed in claim 11, comprising:
   (d) a test strip comprising one or more immobilised nematode CarLA L3 antigens: and
   (e) a reagent comprising a second labelled antibody: and
13. A kit as claimed in claim 11 or 12, wherein the sample container is for saliva.
14. A kit as claimed In claim 11 or 12, wherein the collection means is for the collection of saliva.
15. A kit as claimed in claim 11, further comprising instructions for use. A as in of
16. A kit may as claimed In any one of claims 11 or 15, further comprising detection means to detect one or more labelled antigens.
17. A kit as claimed in claim 12, further comprising detection moans to detect a second labelled antibody.
18. A method of diagnosing nematode Infection in an ungulate animal comprising the steps:
   (a) obtaining a mucus sample from the ungulate; and
   (b) analysing the sample for the presence of an antibody against one or more nematode CarLA L3 antigens.
      whereby the mucus sample is selected from a sample from the nose, mouth, throat, rectal cavity or vagina.
19. A method of determining nematode resistance in an ungulate by assaying a mucus sample from the ungulate for the presence of an antibody specific for one or more CarLA L3 antigens, comprising contacting the sample with one or more antigens selected from the group consisting of:
   (a) a surface antigen on *C.curtical* which runs at substantially 46 kDa and at substantially 22kDa on SDS PAGE gel under reducing conditions;
   (b) a surface antigen on *N.spathiger* which runs at substantially 22kDa on SDS PAGE gel under reducing conditions;
   (c) a surface antigen on *H.contortus* which runs at substantially 35kDa on SDS PAGE gel under reducing conditions: and
   (d) a surface antigen on *O.circumcineta* which runs at substantially 38-39kDa on SDS PAGE gel under reducing conditions;
   (e) a surface antigen on *T.axel* or *T.vitrinus* which runs at substantially 35kDa on SDS PAGE gel under reducing conditions;
   (f) a surface antigen on *O.osiertagi* which runs at substantially 30-45kDa on SOS PAGE gel under reducing canditions;
   (g) a surface antigen on *C.oncophera* which runs at substantially 20 kDa and at substantially 45 kDa on SDS PAGE gel under reducing conditions;
   (h) a surface antigen on *N.brasiliensis* which runs at substantially 9 kDa and at subutantially 12 kDa on SDS PAGE gel under reducing conditions: and
   (i) a surface antigen on *D.eckertl* which runs at substantially 30 kDa an SDS PAGE gel under reducing conditions,
      and measuring the presence or absence of antibody/antigen complex In the sample,
      whereby the presence of the complex is indicative of nematode resistance In the ungulate, and wherein the mucus sample is selected from a sample from the nose, mouth, throat, rectal cavity or vagina.
20. A method of diagnosing nematode infection as claimed in claim 18, or a method of determing nematode resistance as claimed in claim 19, wherein the mucus sample is saliva.

### Brief Description of the Drawings

The invention will now be described in more detail with reference to the accompanying drawings in which:
- Figure 1:: shows a preferred saliva sampling set-up for use in the invention;
- Figure 2:: shows nematode worms labelled to show the presence of CarLA on larval surface;
- Figure 3:: shows CarLA antibody response in sheep compared with FEC;
- Figure 4:: shows CarLA antibody response in Highlander and Coopworth flocks over 24 weeks grazing on nematode infected pasture (the horizontal arrow shows the best time to sample saliva; and the vertical arrow shows the time Highlander ewes were placed on "clean" pasture); and
- Figure 5:: shows an improved saliva centrifugation set-up.

### Detailed Description

The present invention provides a simple efficient test for determining intestinal parasite resistance in ungulate animals.

The test utilises mucus as the biological test sample for the first time. The mucus sample may comprise a sample from the nose, mouth, throat, rectal cavity or vagina of the animal. Preferably, the sample is saliva.

Mucus is not a common biological test sample for detecting the presence of antibodies as it poses numerous problems. One problem is that the mucus is viscous and difficult to work with and generally requires dilution. As antibodies present in the mucus, and in particular saliva, usually originate from serum, they are already present at low concentrations and, after dilution, may not be detectable. In addition, the mucus also contain numerous enzymes, such as proteases, that can digest any antibody present in the sample, making detection even more difficult.

The present invention shows for the first time that antibodies specific against surface coat antigens of nematode L3 are detectable in the saliva of resistant animals, and further that, surprisingly, positive saliva antibody responses were better predictors of parasite resistance in sheep than antibodies measured in serum.

Collection of saliva is non-invasive and can be performed by untrained personnel, unlike blood collection. Saliva collection can be carried out by a single person and is highly efficient (20-25 seconds per animal) and is more likely to be performed on-farm than more complex blood sampling. Thus, the present invention offers numerous advantages over known sampling and testing methods.

It is expected that antibodies specific against surface coat antigens of nematode L3 will be detectable in mucus samples other than saliva of resistant animals, such as in fluid secretions from the nose, throat, rectal cavity and vagina. A skilled worker could easily obtain such samples and test for the presence of L3 antigens to determine parasite resistance according to the method of the present invention.

A method of selecting ungulates that are genetically resistant to one or more nematode worm
infections, said method comprising the steps:
obtaining a sample of mucus from sald ungulate;
testing the sample for the presence of an antibody against the carbohydrate protective coat antigen (CarLA) present on the third Larval stage (L3) of said one or more nematode worms;
and
segregating and selecting ungulates that test positive for the antibody In step (b),
wherein the mucus sample comprises mucosal fluid from the nose, mouth, throat, rectal cavity or vagina.

Preferably the sample is saliva.

The presence of the antibody in the sample is indicative of an ungulate that is genetically resistant to nematode worm infections.

ungulates that are not positive are animals that are less nematode worm resistant. These animals are of low economic value. That value may be increased by these animals being subjected to a differential drenching regime, parasite management regimes or they may be culled.

The term "comprising" as used in this specification and claims means 'consisting at least in part of', that is to say when interpreting statements in this specification and claims which include that term, the features, prefaced by that term in each statement, all need to be present but other features can also be present.

The saliva sample may be obtained by taking a swab from inside of the cheek of the animal. For example, this may be achieved by placing a dental cotton roll, held between forceps, between the inside of the cheek and jaw and gently massaging the inside of the animal's cheek for about 7-15 seconds.

The swab containing saliva sample can then be placed inside a sample container that is appropriately labelled so that it is traceable back to the specific animal. The sample container may contain a preservative, or the sample may be frozen or freeze dried to prevent the sample from deteriorating due to degradation by enzymes and/or bacteria present in the saliva.

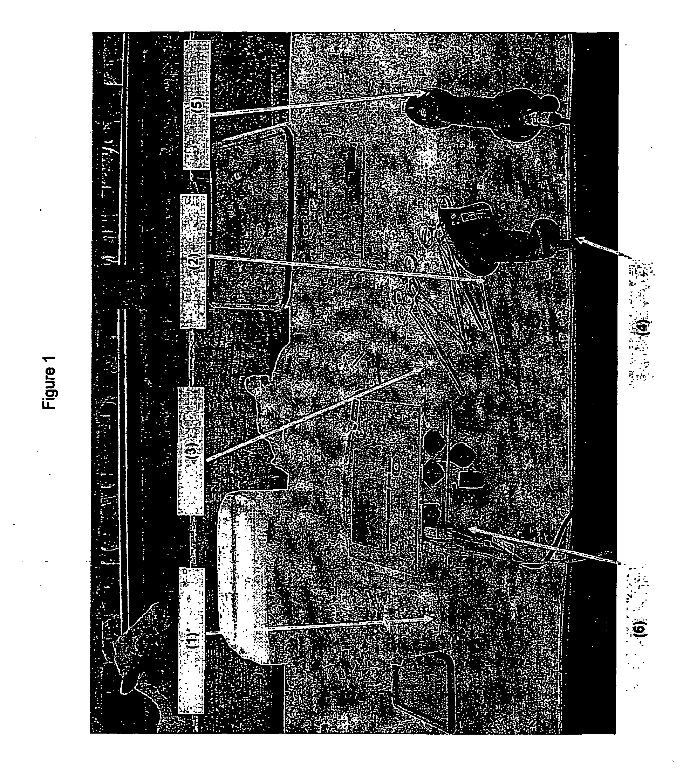

For example, Figure 1 shows a preferred saliva sampling set up whereby, cotton rolls (1), held between forceps (2) are placed inside bar coded vials (3) once an animal's saliva sample has been taken. The barcode on the vial is read using a barcode reader (4) and the animal identity tag read using an EID reader (5). The EID and barcode are matched on a data logger such as a Tru-Test XR3000 system or any other suitable Personal Digital Assistant (PDA) system (6).

The saliva is collected by centrifugation of the cotton roll in a suitable device that allows separation of saliva from cotton roll and the saliva is diluted into a buffer solution containing a preservative. The diluted saliva is then assayed on plates coated with purified intestinal parasite surface coat antigen and the bound antibody is detected generally as a colour reaction whereby the antigen is labelled with a coloured label; or whereby a second labelled antibody is reacted with a non-labelled antibody/antigen complex (ELISA). The absorbance value is quantitative and represents the amount of antibody present in the saliva. The absorbance above a pre-determined cut-off value is a positive result. The absorbance value can be converted into a breeding value.

The animal to be tested is an ungulate including a sheep, cattle, pig, goat, deer or horse.

Preferably the ungulate is a sheep or cattle and most preferably the ungulate is a sheep. The intestinal parasite infection is caused by nematode worms including *Trichostrongylus colubriformis, Haemonchus contortus, Ostertagia (Teladorsagia) circumcincta, Cooperia curticei, Nematodirus spathiger, Trichostrongylus axei, Trichostrongylus vitrinus, Ostertagia ostertagi, Cooperia oncophera, Nematodirus brasiliensis* and *Dictyocaulus eckerti.*

The antibody is specific for CarLA surface antigen present on the third larval stage (L3) of the worm.

"Antibody" refers to an immunoglobulin molecule able to bind to a specific epitope on an antigen. Antibodies can be a polyclonal mixture or monoclonal. Antibodies can be intact immunoglobulins derived from natural sources or from recombinant sources and can be immunoreactive portions of intact immunoglobulins. Antibodies may exist in a variety of forms including, for example, Fv, Fab, and F(ab).sub.2, as well as in single chains. Single-chain antibodies, in which genes for a heavy chain and a light chain are combined into a single coding sequence, may also be used.

The term "L3" refers to a particular larval stage of development in a nematode life cycle. The basic life cycle consists of seven stages, an egg, four larval stages (L1-L4) and two adult stages. The larval stage must progress to L3 before it is capable of infecting another host. The nematodes moult four times during each life cycle separating each of the larval stage and the larval stage from the early adult stage. This moulting process is taken advantage of in the present invention to target antibodies that bond to surface antigens present on the L3 nematode.

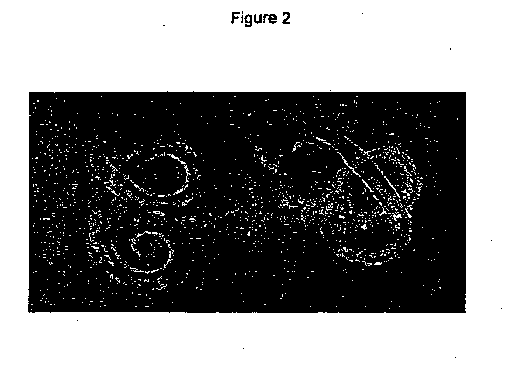

The surface antigen is the carbohydrate protective coat (CarLA) present on nematode L3. This surface coat is shed 4 to 7 days after infection of the host. Animals that are resistant to nematodes produce antibodies to CarLA. Figure 2 shows L3 nematode worms which have been labelled to show CarLA on the larval surface.

The antibody to be detected may comprise monoclonal antibody mAb PAB-1 deposited at ATCC on 24 January 2002, as accorded accession PTA-4005, which binds specifically to CarLA surface antigen or nematode L3.

The antibody may, as described above, be detected using an ELISA assay, or by any other suitable detection method such as immunoblot, western blot, dot blot, agglutination, RIA, and the like.

For a review of immunological an immunoassay procedures in general, see Basic and Clinical Immunology 7th Edition (D. Stite and A. Terr ed.) 1991. The immunoassays of the present invention can be performed in any of several configurations, which are reviewed extensively in Enzyme Immunoassay, E.T. Maggio, ed., CRC Press, Boca Raton, Fla, (1980); "Practice and Theory of Enzyme Immunoassays," P. Tijssen, Laboratory Techniques in Biochemistry and Molecular Biology, Elsevier Science Publishers B.V. Amsterdam (1985). For instance, the antibodies and antigens disclosed here are conveniently used in ELISA, immunoblot analysis and agglutination assays.

In brief, immunoassays to measure anti-CarLA antibodies can be either competitive or noncompetitive binding assays. In competitive binding assays, the sample analyte (e.g., anti-CarLA antibodies) competes with a labelled analyte (e.g., anti-CarLA monoclonal antibody) for specific binding sites on a capture agent (e.g., isolated CarLA antigen) bound to a solid surface. The concentration of labelled analyte bound to the capture agent is inversely proportional to the amount of free analyte present in the sample.

Noncompetitive assays are typically sandwich assays, in which the sample analyte is bound between two analyte-specific binding reagents. One of the binding agents is used as a capture agent and is bound to a solid surface. The second binding agent is labelled and is used to measure or detect the resultant complex by visual or instrument means.

A number of combinations of capture agent and labelled binding agent can be used. For instance, an isolated CarLA antigen can be used as the capture agent and labelled anti-CarLA antibodies specific for the constant region of CarLA antibodies can be used as the labelled binding agent. Alternatively, the anti-CarLA antibodies can be the capture agent and the antigen can be labelled.

Various components of the assay, including the antigen and anti-CarLA antibody, may be bound to a solid surface. Many methods for immobilising biomolecules to a variety of solid surfaces are known in the art. For instance, the solid surface may be a membrane (e.g., nitrocellulose), a microtiter dish (e.g., PVC or polystyrene) or a bead. The desired component may be covalently bound or noncovalently attached through non-specific bonding.

Alternatively, the immunoassay may be carried out in liquid phase and a variety of separation methods may be employed to separate the bound labelled component from the unbound labelled components. These methods are known to those of skill in the art and include immunoprecipitation, column chromatography, adsorption, addition of magnetisable particles coated with a binding agent and other similar procedures.

An immunoassay may also be carried out in liquid phase without a separation procedure. Various homogeneous immunoassay methods are now being applied to immunoassays for protein analytes. In these methods, the binding of the binding agent tot the analyte causes a change in the signal emitted by the label, so that binding may be measured without separating the bound from the unbound labelled component.

Western blot (immunoblot) analysis can also be used to detect the presence of antibodies to CarLA in the sample. This technique is a reliable method for confirming the presence of antibodies against a particular antigen in the sample. The technique generally comprises separating antigens by gel electrophoresis on the basis of molecular weight, transferring the separated antigens to a suitable solid support, (such as a nitrocellulose filter, a nylon filter, or derivatised nylon filter), and incubating the sample with the separated antigens. This causes specific target antibodies present in the sample to bind their respective antigens. Target antibodies are then detected using labelled CarLA antibodies.

The immunoassay formats described above employ labelled assay components. The label can be in a variety of forms. The label may be coupled directly or indirectly to the desired component of the assay according to methods well known in the art. A wide variety of labels may be used. The component may be labelled by any one of several methods. Traditionally radioactive label incorporating ³H, ¹²⁵I, ³⁵S, ¹⁴C, of ³²P was used. Non-radioactive labels include ligands which bind to labelled antibodies, fluorophores, chemiluminescent agents, enzymes, and antibodies which can serve as specific binding pair members for a labelled ligand. The choice of label depends on sensitivity required, ease of conjugation with the compound, stability requirements, and available instrumentation.

Enzymes of interest as labels will primarily be hydrolases, particularly phosphatases, esterases and glyycosidases, or oxidoreductases, particularly peroxidases. Fluorescent compounds include, fluorescein and its derivatives, fhodamine and its derivatives, dansyl, umbelliferone, etc. Chemiluminescent compounds include luciferin, and 2-3-dihydrophthalazinediones, e.g. luminal. For a review of various labelling or signal producing systems which may be used, see U.S. pat. No. 4,391,904.

Non-radioactive labels are often attached by indirect means. Generally, a ligand molecule (e.g., biotin) is covalently bound to the molecule. The ligand then binds to an anti-ligand (e.g., streptavidin) molecule which Is either Inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labelled, naturally occurring anti-ligands. Alternatively, any haptenic or antigenic compound can be used In combination with an antibody. Alternatively gold particles adsorbed with antibodies could be used as would be appreciated by a skilled worker.

Some assay formats do not require the use of labelled components. For instance, agglutination assays can be used to detect the presence of the target antibodies. In this cause, antigen-coated particles are agglutinated by samples comprising the target antibodies. In this format, none of the components needs to be labelled and the presence of the target antibody is detected by simple visual inspection.

The invention further provides a method for detecting the presence of antibodies that are specific for an L3 CarLA surface antigen present on nematode worms in a mucus sample of an ungulate, said method comprising the steps:
(a) obtaining a sample of mucus, from said ungulate comprising mucosal fluid from the nose, mouth, throat, rectal cavity or vagina.
(b) contacting the sample with the L3 CarLA surface antigen, thereby forming an antibody/antigen complex; and
(c) detecting the presence or absence of the complex;
whereby the presence of said antibodies is indicative of an ungulate that is genetically resistant to nematode worm infection.

The antigen may comprise one or more surface antigens on nematode L3 selected from the group consisting of:
(a) a surface antigen on *C.curticei* which runs at substantially 46 kDa and at substantially 22kDa on SDS PAGE gel under reducing conditions;
(b) a surface antigen on *N.spathiger* which runs at substantially 22kDa on SDS PAGE gel under reducing conditions;
(c) a surface antigen on *H.contortus* which runs at substantially 35kDa on SDS PAGE gel under reducing conditions; and
(d) a surface antigen on *O.circumcincta* which runs at substantially 35-39kDa on SDS PAGE gel under reducing conditions;
(e) a surface antigen on *T.axei* or *T.vitrinus* which runs at substantially 35kDa on SDS PAGE gel under reducing conditions;
(f) a surface antigen on *O.ostertagi* which runs at substantially 30-45kDa on SDS PAGE gel under reducing conditions;
(g) a surface antigen on *Concophera* which runs at substantially 20 kDa and at substantially 45 kDa on SDS PAGE gel under reducing conditions;
(h) a surface antigen on *N.brasiliensis* which runs at substantially 9 kDa and at substantially 12 kDa on SDS PAGE gel under reducing conditions; and
(i) a surface antigen on *D.eckerti* which runs at substantially 30 kDa on SDS PAGE gel under reducing conditions.

The antigen may be immobilised on a solid surface and the complex detected by using a label on the antigen which is detectable when the complex is formed. Alternatively, the complex may be detected by using a second labelled antibody which binds to the complex.

The detection is preferably carried out using a test strip comprising immobilised antigen. The test strip is contacted with the sample and the antigen/antibody complex formed if the antibody is present in the sample. Where the antigen is labelled, the label becomes detectable upon the complex being formed. A further step may be required to remove excess sample.

Alternatively, a reagent comprising a second labelled antibody can be added to the test strip and a detectable change induced if the second labelled antibody binds to the complex. A detectable change indicates the presence of the antibody in sample which is associated with parasite resistance in the animal.

The sample is preferably saliva and may be collected as described above before being contacted with the test strip, for example, by dipping the test strip into a diluted saliva sample. Alternatively, the test strip may be modified in some way so that it can be used in place of the cotton roll to collect saliva directly from the animal's oral cavity.

A positive result is indicative of an animal that shows host resistance to intestinal parasites.

Also described is a test strip comprising one or more of the abovementioned nematode L3 surface antigens immobilised thereto.

The present invention also provides a kit for detecting the presence of an antibody specific for a nematode carLA L3 surface antigen, said kit comprising:
(a) a container for a mucus sample;
(b) a reagent comprising a labelled nematode CarLA L3 antigens and
(c) a mucus sample collection means;
wherein the mucus sample is selected from a sample from the nose, throat, rectal cavity or vagina.

Preferably the container and collection means are for a saliva sample.

The kit of the invention may further comprise instructions for use.

The kit may also comprise detection means to detect a labelled antigen or to detect a labelled second antibody.

The kit may comprise one or more of the nematode L3 antigens listed above.

The invention also provides a method for diagnosing nematode infection in an ungulate animal, comprising the steps:
(a) obtaining a mucus samples from the ungulate; and
(b) analysing the sample for the presence of an antibody against nematode CarLA L3 antigen
wherein the mucus sample is selected from a sample from the nose, throat, rectal cavity or vagina.

The sample is preferably saliva which may be collected and analysed by any of the methods described above.

The antigen may comprise one or more nematode CarLA L3 antigens selected from the group consisting of:
(a) a surface antigen on C.*curticei* which runs at substantially 46 kDa and at substantially 22kDa on SDS PAGE gel under reducing conditions;
(b) a surface antigen on *N.spathiger* which runs at substantially 22kDa on SDS PAGE get under reducing conditions;
(c) a surface antigen on *H.contortus* which runs at substantially 35kDa on SDS PAGE gel under reducing conditions: and
(d) a surface antigen on *O.circumcincta* which runs at substantially 35-39kDa on SDS PAGE gel under reducing conditions:
(e) a surface antigen on *T.axei* or *T.vitrinus* which runs at substantially 35kDa on SDS PAGE gel under reducing conditions:
(f) a surface antigen on O. *ostertagi* which runs at substantially 30-45kDa on SDS PAGE gel under reducing conditions;
(g) a surface antigen on *C.oncophera* which runs at substantially 20 kDa and at substantially 45 kDa on SDS PAGE gel under reducing conditions:
(h) a surface antigen on *N.brasiliensis* which runs at substantially 9 kDa and at substantially 12 kDa on SDS PAGE gel under reducing conditions; and
(i) a surface antigen on *D.eckerti* which runs at substantially 30 kDa on SDS PAGE gel under reducing condttions.

The antigen may be immobilised on a solid surface and the complex detected by using a label on the antigen which is detectable when the complex is formed. Alternatively, the complex may be detected by using a second labelled antibody which binds to the complex, as described above.

The sample may be collected and assayed by any one of the methods described above.

The invention consists in the foregoing and also envisages constructions of which the following gives examples only.

### EXAMPLE 1

### Materials and Methods

Lambs grazed on open pasture were exposed to 2-4 cycles of nematode challenge whereby the FEC was allowed to build up to 800-1000 epg before the animals were faecal sampled and drenched.

All lambs had saliva collected by using the saliva sampling set-up shown in figure 1, whereby a cotton roll (1), held by forceps (2) was inserted into the mouth of the lamb between the cheek and the jaw, and the cheek massaged for around 15 seconds. The cotton roll was placed in a bar-coded vial (3), and the animal's identity tag scanned using an EID reader (5), and the bar-coded vial read by a barcode reader (4). The EID and bar-coded sample were matched using a Tru-Test XR3000 system. This set-up allows the sample to be directly traced to the animal from which it was taken.

The saliva was then collected by centrifuging the cotton roll and the saliva diluted with a buffer containing a preservative. The diluted saliva was then contacted with a multi-well plate coated with purified, colour-labelled CarLA antigen. Once the antibody in the saliva sample bound to the CarLA antigen, the complex formed was detected using appropriately labelled anti-antibodies, anti-ovine IgG1 or anti-ovine IgA antibodies labelled with horse-raddish-peroxidase or further detected with labelled anti IgG conjugate. The chemical reaction produced using peroxidise reactions was detected using a spectrometer capable of measuring the absorbance of the coloured label (reaction).

Saliva and FEC sampling and testing occurred regularly between 13 and 110 days post weaning.

### Results

18% of the lambs tested positive for CarLA antibodies in their saliva. The faecal egg count of these animals was 32% lower than the CarLA negative animals.

Animals were tested again approximately five to six weeks later, and 61% of the lambs tested were positive for CarLA antibodies in their saliva. Of these animals, their FEC was 22% lower than CarLA negative animals.

The animals which had tested positive for CarLA were re tested after a further 5 to 6 weeks and had a 49.3% lower FEC than those animals that had previously tested negative.

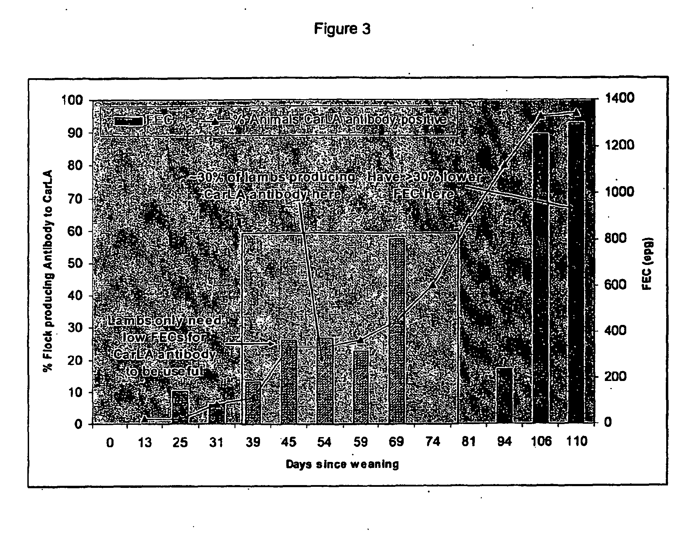

These results are shown in Figure 3.

### Discussion

Both saliva IgG1 and IgA to CarLA was significantly correlated with reduced FEC. Serum antibody levels were also measured in blood samples that were taken at the same time as the saliva samples. Surprisingly, the serum antibody levels did not correlate as well as saliva antibody levels with FEC (data not shown).

The results also show that the present CarLA antibody detection test is useful in selecting host protective lambs, even when the lambs are receiving a preventive drench.

The sampling can be carried out at any time from about 6 to 11 weeks or more post weaning, depending on the level of challenge in pasture.

These results demonstrate that antibodies specific against CarLA can be detected in the saliva of animals, and such detection can be used to select for Host Resistance.

### EXAMPLE 2

### Materials and Methods

Rissington Breedline Highlander lambs (n=299) were grazed on nematode infected pasture and randomly sampled (n=30) for saliva, serum and faeces at weekly intervals. The whole flock was then sampled for saliva, serum and FEC. At this time the animals were weighed and dag scored. (Sampling 1). This was repeated five to eight weeks later. (Sampling 2). All weekly and sampling 1 and 2 saliva and serum samples were assayed for CarLA specific IgG1 IgE and IgA by EUSA.

### Results

Approximately 40% of the flock tested positive for CarLA IgG1 antibodies in their saliva. This increased significantly by the end of the trial so that all the animals were producing detectable levels of IgG1 antibody to CarLA in saliva. Compared to the results in example 1, the increase in reactivity was probably due to the stronger and earlier nematode challenge.

The saliva IgG1 and IgA results in both sampling 1 and 2 correlated with reduced FEC at the second sampling (FEC2). In particular, saliva IgG1 at Sampling 1 (64.1% of flock) predicted 26.6% reduction in FEC at FEC2, and at Sampling 2 (60.2% of flock) predicted 25.8% reduction in FEC. Saliva IgA was also correlated with reduced FEC. Saliva IgA at sampling 1 (64.0% of flock) predicted 29.7% reduction in FEC at FEC2, and at Sampling 2 (35.8% of flock) predicted 24.7% reduction in FEC.

Serum IgG1, IgA and IgE levels did not correlate with reductions in FEC as shown in table 1, below.

**Table 1: mean reduction in FEC for all weekly sampling**

| | **Saliva IgG1** | **Serum IgG1** | **Saliva IgA** | **Serum IgA** | **Serum IgE** |
|---|---|---|---|---|---|
| FEC 2 | 35.3± 12.1% | 24.7± 20.1% | 28.8± 14.4% | 29.5± 25.8 | 9.3± 37.3% |

| | | | | | |
|---|---|---|---|---|---|
| Saliva IgG1 and IgA correlated with the best predictions of FEC reduction and had the lowest variability. | | | | | |

### Discussion

Both saliva IgG1 and IgA to CarLA was significantly correlated with reduced FEC and was able to predict reduced FEC using either the weekly sampling or the Sampling 1 or Sampling 2 data. The fact that saliva gave better results than serum suggests that CarLA specific antibodies are produced in the oral cavity to complement the antibody in oral fluid derived from serum through a transudation process.

The results of this trial confirm the results of example 1, namely that antibodies specific against CarLA can be detected in saliva of animals, and can be used to select for Host Resistance. The results further confirmed that the saliva testing was superior to the serum testing.

### EXAMPLE 3

### Materials and Methods

Rissington Breedline Highlander lambs (n=315) were saliva sampled at weaning [Sampling 1] before being grazed on open pasture. Weekly random sampling of the flock (n=50) for saliva and faecal egg count samples (when appropriate) began at week 2. The whole flock was sampled for saliva at week 8 [Sampling 2] and at week 16 [Sampling 3].

The whole flock was also sampled for FEC at week 8 (FEC1a, mean epg 785 (arithmetic)) and week 9 (FEC1b, mean epg 821). Following sampling at week 9, the lambs were drenched and crutched.

A second round of sampling for FEC occurred at week 17 (FEC2a, mean epg 332), week 18 (FEC2b, mean epg 330) and week 19 (FEC2c, mean epg 365). Following sampling at week 18, the lambs were weighed arid dag scored. All weekly and Sampling 1, 2 & 3 saliva samples were assayed for CarLA specific IgG1 and IgA by ELISA.

Similar saliva and FEC sampling was also carried out on recorded flocks of Perendale, Coopworth and Romney lambs.

Saliva samples were collected and prepared for CarLA analysis as described above in example 1.

### Results

Saliva IgA CarLA specific antibodies were able to predict a significant reduction in faecal egg count at FEC2 in Highlander, Coopworth and Perendale flocks. Saliva IgG1 CarLA specific antibodies were able to predict significant reduction in FEC in the Highlander, Coopworth and Perendale flocks, but not in the Romneys. The reason for this lack of prediction response in the Romney flock for saliva IgA and IgG1 was not apparent (results not shown).

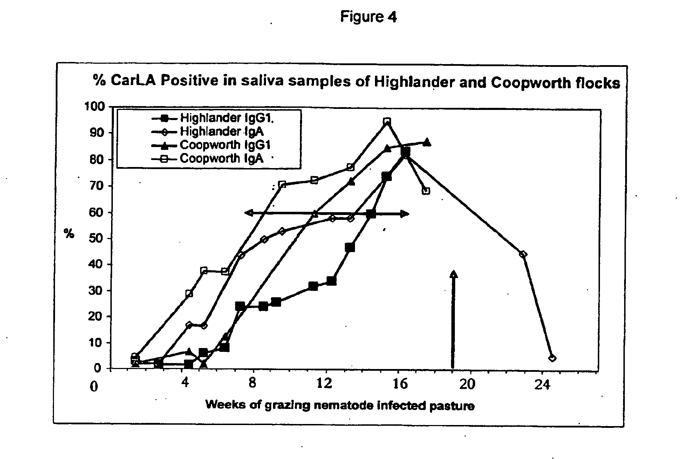

Further analysis of the results of IgA and IgG1 for Highlander and Coopworth lambs showed that the best time to sample saliva is between 8 and 16 weeks after weaning. (Figure 4).

Genetic analysis of the results showed that the CarLA IgA and IgG1 responses were heritable with overall Heritabilities of 0.29 and 0.20 respectively. The genetic correlation of CarLA antibody with FEC was between -0.27 and -0.51 for IgG1 and -0.23 and -0.72 for IgA.

Analysis of the phenotypic relationship between CarLA antibody in saliva and breech soiling ('dags') or productivity as measured at weight or weight gain showed that any adverse correlation if significant were weak. In contrast there were consistently unfavourable correlations between FEC and productivity traits.

### Larval culture

Faecal samples for Highlander ewes were taken at FEC2c and larvae cultured by known methods. The results are shown in table 2, below.

**Table 2**

| **Nematode** | **Mean % (n=5)** |
|---|---|
| Heamonchus | 34% |
| Ostertagia | 42% |
| Trichostrongylus | 9% |
| Cooperia | 14% |
| Long Tail | 1% |

The larval cultures of FEC2c showed that *Trichostrongylus* made up less than 10% of the larvae that developed from eggs excreted by the Highlander ewes. However, IgA responses to *T. colubriformis* CarLA measured eight weeks before FEC2c sampling and one week prior to FEC2c sampling predicted significant reductions in FEC at FEC2 sampling so that this result was consistent with the saliva test prediction.

### EXAMPLE 4

In order to extend the carbohydrate larval surface antigen (CarLA) validation studies to different management types and breeds, a flock of 289 Merino lambs was sampled for faecal egg count (FEC) and saliva, after weaning. FEC (strongyles) had not built up to a severe level by the time of sampling approximately 5 months after weaning and the average of back-transformed FEC was 197 eggs/g. *Nematodirus* (NEM) eggs were counted at the same time, and their average was 101 eggs/g (back-transformed log egg count). Previous to this sampling, the flock had experienced an acute NEM outbreak three months earlier at which point the flock was drenched therefore we are likely to be measuring the ability of the sheep to respond to a challenge infection. The NEM infection was more significant biologically than the strongyle infection, and this is shown in the correlations between egg counts and saliva concentrations. For NEM, correlations with salivary IgG1 and IgA (units) were significant (P < 0.0378 and P < 0.0001, respectively). Higher saliva concentrations indicated lower NEM egg counts. Genetic analysis of the results showed that IgG1 response to CarLA was significantly heritable (0.657 ± 0.286) but apparently not IgA unlike the findings from other larger data sets and breeds. Few genetic correlations had small enough standard errors to provide for useful interpretations, except NEM x IgG1 (-0.716 ± 0.216) which was significantly negative, as expected from other breeds of data analysed. The genetic correlation estimate for NEM x IgA was also significant, although the estimate was outside the upper bound of 1.0 expected, so that the result may or may not be biologically significant.

### Discussion

Saliva IgG1 and IgA was significantly correlated with reduced FEC in a number of different sheep breeds.

Saliva IgG1 and IgA antibody response was both heritable and genetically correlated with reduced FEC suggesting that selection of animals based on these tests would results in reducing animal FECs

Sampling is best carried out between 8 and 16 weeks after grazing on nematode infested pasture.

There were no strong unfavourable correlations between CarLA antibody (IgA or IgG1) responses and traits such as weight gain or breech soiling.

These results demonstrate that antibodies specific against CarLA can be detected in the saliva of a number of different breeds of sheep and used to predict a reduction in FEC in individual animals. Such a test is therefore useful to select for Host Resistance. This test is particularly useful to select for resistance to *Trichostrongulus* nematodes.

### EXAMPLE 5

### Improved saliva processing and assaying

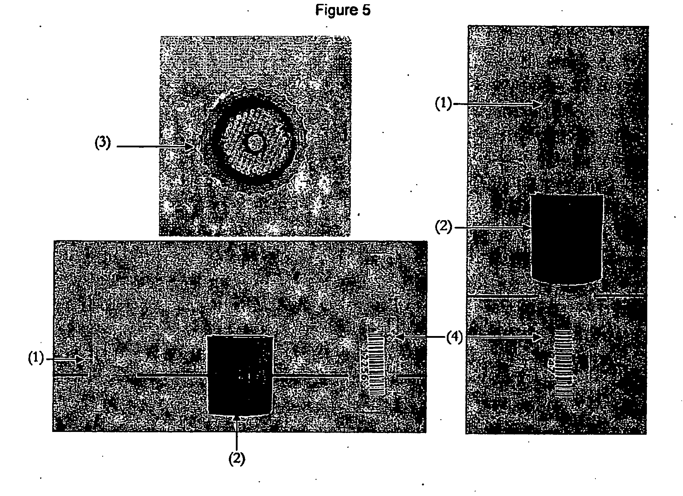

Extraction of saliva from cotton roll can be aided by centrifuging cotton roll after saliva has been collected. As illustrated in Figure 5, cotton roll is transferred to a dean 5ml vial (1). This is attached to an adapter (2) (Lab-Serv) that has an open plastic filter (3). The original labelled vial (4) is attached to the other side of the adaptor. The whole set-up is place in a centrifuge with cotton-roll end upwards. This is centrifuged for 2-5 min at 2000g in an appropriate centrifuge. Extracted saliva is retained in the labelled vial, ready for dilution for assaying for CarLA specific antibody.

The inventors have also found that the use of ELISA plates coated with CarLA and stored at 4°C as opposed to storage at -20°C results in more consistent assay results.

In addition, using a reference standard method where simple linear regression analysis of logarithmic or logit-logarithmic transformed data obtained from titration of reference standard made of serially diluted pooled CarLA antibody positive serum, results in more consistent assay results than using an optical density index method.

### EXAMPLE 6

Further sampling on recorded flocks of various breeds including Romney, Coopworth, Merino and Highlander is to be carried out to more accurately assess the ability of a CarLA antibody to predict reduced FEC in these breeds. In particular recorded flocks experiencing *Haemonchus contortus* infection will be analysed for their CarLA antibody response in relation to reduced FEC. Previously sampled breeds of Romney, Highlander, Coopworth and Merino types will be further sampled to increase sample size for assessing the heritability of CarLA antibody and genetic correlations with FEC and other parameters. It is expected that saliva IgA and IgGI will accurately predict a reduction in FEC in individual animals which will be useful to select for Host Resistance.

### EXAMPLE 7

### Materials and Methods

Bovine calves grazing on open pasture will be exposed to 2-4 cycles of nematode challenge whereby the FEC will build up to approximately 800-1000 epg before the animals are faecal sampled and drenched.

Saliva from the bovine calves will be collected as for sheep species and tested for the presence of CarLA antigen as described above.

### Results

It is expected that the bovine calves will test positive for CarLa antibodies in their saliva. Specifically, CarLA from the bovine nematodes *O.ostertagi.* and *C.oncophera* are likely to be detected.

The test of the present invention will be useful in selecting Host Resistant bovine animals.

### Comparative EXAMPLE 8

### Breeding Host Resistant Animals

The results of the IgA concentration of grazing animals as described above, can be transformed onto a logarithmic scale, and parasite-resistance Breeding Values (BVs) calculated from log IgA, using "animal-model Restricted Maximum Likelihood" statistical procedures as published by Gilmour, A.R. (1997). These procedures use log IgA records for all known pedigrees. They take account of the fact that the IgA trait is inherited, so that records from relatives (e.g., animals with a common sire, or twins with two common parents) become useful in assisting with the prediction of an individual's most likely merit as a parent (its BV). From the parasite-resistance BV results, all animals of one sex are ranked, and those with the best BVs are used as parents of the next generation. The BV ranking list may include recorded and unrecorded animals, with the BVs for the unrecorded animals depending entirely on the data from recorded relatives.

It is not the intention to limit the scope of the invention to the abovementioned example only. As would be appreciated by a skilled person in the art, many variations are possible without departing from the scope of the invention as claimed in the appended claim set.

### Industrial Application

The saliva diagnostic test of the present invention can be used to select for animals that are genetically resistant to intestinal parasites, particularly nematode worms, to reduce the use of anthelmintics.

### References

Harrison GB, Pulford HD, Hein WR, Barber TK, Shaw, RJ, McNeill M, Wakefield SJ, Shoemaker CB. Immune rejection of Trichostrongylus colubriformis in sheep; a possible role for intestinal mucus antibody against an L3-specific surface antigen. Parasite Immunology 2003, 25(1): 45-53.

Gilmour, AR. ASREML for testing fixed effects and estimating multiple trait variance components. Proceedings of the Association for the Advancement of Animal Breeding and Genetics 1997,12:386-390.

## Claims

1. A method of selecting ungulates that are genetically resistant to one or more nematode worm infections, said method comprising the steps:
(a) obtaining a sample of mucus from said ungulate
(b) testing the sample for the presence of an antibody against the carbohydrate protective coat antigen (CarLA) present on the third larval stage (L3) of said one or more nematode worms; and
(c) segregating and selecting ungulates that test positive for the antibody In step (b), wherein the mucus sample comprises mucosal fluid from the nose, mouth, throat, rectal cavity or vagina.

2. A method as claimed in claim 1, wherein the mucus sample comprises saliva.

3. A method as claimed in claim 1, wherein the one or more nematode worms are selected from the group consisting of *Trichostrongylus colubriformis, Haemonchus confortus, Ostertagia (Teladorsagia) circumcincta, Cooperia curticel, Nematodirus spathiger, Trichostrongylus axei. Trichostrongylus vitrinus, Osteriagia ostertagi, Cooperia oncophera, Nematodirus brasslliensis* and *Dictyocaulus eckerti.*

4. A method as claimed in any one of claims 1-8, wherein the antibody comprises monoclonal antibody mab PAB-1, (ATCC accession no. PTA-4005), which specifically binds to the CarLA surface antigen on nematode L3.

5. A method for detecting the presence of antibodies that are specific for an L3 CarLA surface antigen present on one or more species of nematode worms In a mucus sample from an ungulate, said method comprising the steps:
(a) obtaining a sample of mucus from said ungulate comprising mucosal fluid from the nose, mouth, throat, rectal cavity or vagina.
(b) contacting said sample with the L3 CarLA surface antigen, thereby forming an antibody/antigen complex; and
(c) detecting the presence or absence of the complex;
wherein the presence of said antibody/antigen complex is indicative of an ungulate that is genetically resistant to nematode worm infection.

6. A method as claimed in claim 5, wherein the mucus sample is salive.

7. A method as claimed in claim 5 or 6, wherein the antigen is selected from one or more nematode L3 CarLA antigens selected from the group consisting of;
(a) a surface antigen on *C.curticel* which rune at substantially 46 kDa and at substantially 22kDa on SDS PAGE gel under reducing conditions;
(b) a surface antigen on *N.spathiger* which runs at substantially 22kDa on SDS PAGE gel under reducing conditions;
(c) a surface antigen on *H.contortus* which runs at substantially 35kDa on SDS PAGE gel under reducing conditions; and
(d) a surface antigen on *O.ciroumoincta* which runs at substantially 38-89kDa on 9DS PAGE gel under reducing conditions;
(e) a surface antigen on *T.axel*or *T.vitrinus* which runs at substantially 36kDa on SDS PAGE gel under reducing conditions;
(f) a surface antigen on *O.ostertagl* which runs at substantially 30-46kDa on SDS PAGE gel under reducing conditions;
(g) a surface antigen on *C*.ancophers which runs et substantially 20 kDa and at substantially 48 kDa on 8D8 PAGE gel under reducing condidons;
(h) a surface antigen on *N:brasiliensis* which runs at substantially 9 kDa end at substentially 12 kDa on SDS PAGE gel under reducing conditions; and
(i) a surface antigen on D.eckertl which runs at substantially 30 kDa on SDS PAGE gel under reducing conditions.

8. A method as claimed In claim 7, wherein the antigen is immobilised on a solid surface and the complex detected by using a label on the antigen which is detectable when the complex is formed.

9. A method as claimed in claim 8, wherein the complex is detected by using a second labelled antibody which binds to the complex.

10. A method as claimed in claim 8 or 9, wherein the detection is carried out using a test strip comprising one or more immobilised antigens.

11. A kit for detecting the presence of an antibody specific for one or more antibody for one more nematode CarLA L3 surface antigene, said kit comprising:
(a) a container for a mucus sample:
(b) a reagent comprising one or more labelled nematode CarLA L3 antigens; and
(c) a mucus sample collection means;
wherein the mucus sample is selected from a sample from the nose, mouth, throat, rectal cavity or vagina.

12. A kit as claimed in claim 11, comprising
(d) a test strip comprising one or more immobilised nematode CarLA L3 antigene; and
(e) a reagent comprising a second labelled antibody.

13. A kit as claimed in claim 11 or 12, wherein the sample container is for saliva.

14. A kit as claimed in claim 11 or 12, wherein the collection means is for the collection of

15. A kit as claimed in claim 11, further comprising instructions for use.

16. A kit as claimed in any one of claims 11 to 15, further comprising detection means to detect one or more labelled antigens.

17. A kit as claimed in claim 12, further comprising detection moans to detect a second labelled antibody.

18. A method of diagnosing nematode infection in an ungulate animal comprising the steps:
(a) obtaining a mucus sample from the ungulate; and
(b) analysing the sample for the presence of an antibody against one or more nematode Car LA L3 antigens.
whereby the mucus sample is selected from a sample from the nose, mouth, throat, rectal cavity or vagina.

19. A method of detarmining nematode resistance In an ungulate by assaying a mucus sample from the ungulate for the presence of an antibody specific for one or more sample Car LA L3 antigens, contacting the sample with one or more antigens selected from the group consisting of:
(a) a surface antigen on *C.curticel* which runs at substantially 46 kDa and at substantially 22kDa on SDS PAGE gel under reducing conditions;
(b) a surface antigen on *N.spathiger* which runs at substantially 22kDa on SDS PAGE gel under reducing conditions;
(c) a surface antigen on *H.contorius* which runs at substantially 35kDa on SDS PAGE gel under reducing conditions; and
(d) a surface antigen on *O.circumcincta* which runs at substantially 38-39kDa on SDS PAGE gel under reducing conditions;
(e) a surface antigen on *T.axei* or *T.vitrinus* which runs at substantially 35kDa on SDS PAGE gel under reducing conditions;
(f) a surface antigen on *O.ostertagi* which runs at subtantially 30-45kDa on SDS PAGE gel under reducing conditions;
(g) a surface antigen on *C.oncophera* which runs at substantially 20 kDa and at substantially 45 kDa on SDS PAGE gel under reducing conditions;
(h) a surface antigen on *N.brasillensis* which runs at substantially 9 kDa and at substantially 12 kDa on SDS PAGE gel under reducing conditions; and
(i) a surface antigen on *D.eckerti* which runs at substantially 30 kDa on SDS PAGE gel under reducing conditions,
and measued the presence or absence of antibody/antigen complex in the sample, whereby the presence of the complex is indicative of nematode resistance in the ungulate, and wherein the mucus sample is selected from a sample from the nose, mouth, throat, rectal cavity or vagina.

20. A method of diagnosing nematode infection as claimed in claim 18 or a method of determining nematode resistance as claimed in claim 19.

## Patentansprüche

1. Verfahren zum Selektieren von Huftieren, die gegen eine oder mehrere Nematodenwurm-Infektionen genetisch resistent sind, wobei das Verfahren folgende Schritte aufweist:
(a) Beschaffen einer Schleimprobe von dem Huftier;
(b) Testen der Probe auf die Anwesenheit eines Antikörpers gegen das Kohlenhydrat-Schutzhüllenantigen (CarLA), das auf dem dritten Larvenstadium (L3) des einen Nematodenwurms oder der mehreren Nematodenwürmer vorliegt; und
(c) Separieren und Selektieren von Huftieren, die im Test auf den Antikörper in Schritt (b) positiv sind,
wobei die Schleimprobe Schleimhautfluid aus der Nase, dem Mund, dem Rachen, dem Rektalraum oder der Vagina aufweist.

2. Verfahren wie in Anspruch 1 beansprucht, wobei die Schleimprobe Speichel aufweist.

3. Verfahren wie in Anspruch 1 beansprucht, wobei der eine oder die mehreren Nematodenwürmer ausgewählt werden aus der Gruppe, die besteht aus Trichostrongylus colubriformis, Haemonchus contortus, Ostertagia (Teladorsagia) circumcincta, Cooperia curticei, Nematodirus spathiger, Trichostrongylus axei, Trichostrongylus vitrinus, Ostertagia ostertagi, Cooperia oncophera, Nematodirus brasiliensis und Dictyocaulus eckerti.

4. Verfahren wie in einem der Ansprüche 1 bis 3 beansprucht, wobei der Antikörper den monoklonalen Antikörper mAb PAB-1, (ATCC-Zugangsnummer PTA-4005), aufweist, der spezifisch an das CarLA-Oberflächenantigen auf der L3-Nematode bindet.

5. Verfahren zum Nachweisen der Anwesenheit von Antikörpern, die spezifisch sind für ein L3-CarLA-Oberflächenantigen, das auf einer oder mehreren Spezies von Nematodenwürmern in einer Schleimprobe von einem Huftier vorliegt, wobei das Verfahren folgende Schritte aufweist:
(a) Beschaffen einer Schleimprobe von dem Huftier, die Schleimhautfluid aus der Nase, dem Mund, dem Rachen, dem Rektalraum oder der Vagina aufweist,
(b) in Berührung Bringen der Probe mit dem L3-CarLA-Oberflächenantigen unter Bildung eines Antikörper/Antigen-Komplexes; und
(c) Nachweisen der Anwesenheit oder Abwesenheit des Komplexes;
wobei die Anwesenheit des Antikörper/Antigen-Komplexes auf ein Huftier schließen lässt, das gegen eine Nematodenwurm-Infektion genetisch resistent ist.

6. Verfahren wie in Anspruch 5 beansprucht, wobei die Schleimprobe Speichel ist.

7. Verfahren wie in Anspruch 5 oder 6 beansprucht, wobei das Antigen ausgewählt wird aus einem oder mehreren L3-Nematoden-CarLA-Antigenen, die ausgewählt werden aus der Gruppe, die besteht aus:
(a) einem Oberflächenantigen auf C.curticei, das auf SDS-PAGE-Gel unter reduzierenden Bedingungen bei im Wesentlichen 46kDa und bei im Wesentlichen 22kDa wandert;
(b) einem Oberflächenantigen auf N, spathiger, das auf SDS-PAGE-Gel unter reduzierenden Bedingungen bei im Wesentlichen 22kDa wandert;
(c) einem Oberflächenantigen auf H.contortus, das auf SDS-PAGE-Gel unter reduzierenden Bedingungen bei im Wesentlichen 35kDa wandert; und
(d) einem Oberflächenantigen auf O.circumcincta, das auf SDS-PAGE-Gel unter reduzierenden Bedingungen bei im Wesentlichen 35-39kDa wandert;
(e) einem Oberflächenantigen auf T.axei oder T.vitrinus, das auf SDS-PAGE-Gel unter reduzierenden Bedingungen bei im Wesentlichen 35kDa wandert;
(f) einem Oberflächenantigen auf O.ostertagi, das auf SDS-PAGE-Gel unter reduzierenden Bedingungen bei im Wesentlichen 30-45kDa wandert;
(g) einem Oberflächenantigen auf C.oncophera, das auf SDS-PAGE-Gel unter reduzierenden Bedingungen bei im Wesentlichen 20kDa und bei im Wesentlichen 45kDa wandert;
(h) einem Oberflächenantigen auf N.brasiliensis, das auf SDS-PAGE-Gel unter reduzierenden Bedingungen bei im Wesentlichen 9kDa und bei im Wesentlichen 12kDa wandert; und
(i) einem Oberflächenantigen auf D.eckerti, das auf SDS-PAGE-Gel unter reduzierenden Bedingungen bei im Wesentlichen 30kDa wandert.

8. Verfahren wie in Anspruch 7 beansprucht, wobei das Antigen auf einer festen Oberflächen immobilisiert wird und der Komplex unter Verwendung einer Markierung auf dem Antigen, die nachweisbar ist, wenn der Komplex gebildet wird, nachgewiesen wird.

9. Verfahren wie in Anspruch 8 beansprucht, wobei der Komplex unter Verwendung eines zweiten markierten Antikörpers, der an den Komplex bindet, nachgewiesen wird.

10. Verfahren wie in Anspruch 8 oder 9 beansprucht, wobei der Nachweis unter Verwendung eines Teststreifens, der ein oder mehrere immobilisierte Antigene aufweist, durchgeführt wird.

11. Kit zum Nachweisen der Anwesenheit eines Antikörpers, der spezifisch ist für ein oder mehrere Nematoden-CarLA-L3-Oberflächenantigene, wobei das Kit aufweist:
(a) einen Behälter für eine Schleimprobe;
(b) ein Reagenz, das ein oder mehrere markierte Nematoden-CarLA-L3-Antigene aufweist; und
(c) eine Schleimproben-Sammeleinrichtung;
wobei die Schleimprobe ausgewählt wird aus einer Probe aus der Nase, dem Mund, dem Rachen, dem Rektalraum oder der Vagina.

12. Kit wie in Anspruch 11 beansprucht, aufweisend:
(d) einen Teststreifen, der ein oder mehrere immobilisierte Nematoden-CarLA-L3-Antigene aufweist; und
(e) ein Reagenz, das einen zweiten markierten Antikörper aufweist.

13. Kit wie in Anspruch 11 oder 12 beansprucht, wobei der Probenbehälter für Speichel ist.

14. Kit wie in Anspruch 11 oder 12 beansprucht, wobei die Sammeleinrichtung für das Sammeln von Speichel ist.

15. Kit wie in Anspruch 11 beansprucht, außerdem Bedienungsanweisungen aufweisend.

16. Kit wie in einem der Ansprüche 11 bis 15 beansprucht, außerdem eine Nachweiseinrichtung zum Nachweisen eines oder mehrerer markierter Antigene aufweisend.

17. Kit wie in Anspruch 12 beansprucht, außerdem eine Nachweiseinrichtung zum Nachweisen eines zweiten markierten Antikörpers aufweisend.

18. Verfahren zum Diagnostizieren einer Nematodeninfektion bei einem Huftier, folgende Schritte aufweisend:
(a) Beschaffen einer Schleimprobe von dem Huftier; und
(b) Analysieren der Probe auf die Anwesenheit eines Antikörpers gegen ein oder mehrere Nematoden-CarLA-L3-Antigene,
wobei die Schleimprobe ausgewählt wird aus einer Probe aus der Nase, dem Mund, dem Rachen, dem Rektalraum oder der Vagina.

19. Verfahren zum Bestimmen der Nematodenresistenz bei einem Huftier durch Untersuchen einer Schleimprobe von dem Huftier auf die Anwesenheit eines Antikörpers, der spezifisch ist für ein oder mehrere CarLA-L3-Antigene, aufweisend ein In-Berührung-Bringen der Probe mit einem oder mehreren Antigenen, die ausgewählt werden aus der Gruppe, die besteht aus:
(a) einem Oberflächenantigen auf C.curticei, das auf SDS-PAGE-Gel unter reduzierenden Bedingungen bei im Wesentlichen 46kDa und bei im Wesentlichen 22kDa wandert;
(b) einem Oberflächenantigen auf N.spathiger, das auf SDS-PAGE-Gel unter reduzierenden Bedingungen bei im Wesentlichen 22kDa wandert;
(c) einem Oberflächenantigen auf H.contortus, das auf SDS-PAGE-Gel unter reduzierenden Bedingungen bei im Wesentlichen 35kDa wandert; und
(d) einem Oberflächenantigen auf O.circumcincta, das auf SDS-PAGE-Gel unter reduzierenden Bedingungen bei im Wesentlichen 35-39kDa wandert;
(e) einem Oberflächenantigen auf T.axei oder T.vitrinus, das auf SDS-PAGE-Gel unter reduzierenden Bedingungen bei im Wesentlichen 35kDa wandert;
(f) einem Oberflächenantigen auf O.ostertagi, das auf SDS-PAGE-Gel unter reduzierenden Bedingungen bei im Wesentlichen 30-45kDa wandert;
(g) einem Oberflächenantigen auf C.oncophera, das auf SDS-PAGE-Gel unter reduzierenden Bedingungen bei im Wesentlichen 20kDa und bei im Wesentlichen 45kDa wandert;
(h) einem Oberflächenantigen auf N.brasiliensis, das auf SDS-PAGE-Gel unter reduzierenden Bedingungen bei im Wesentlichen 9kDa und bei im Wesentlichen 12kDa wandert; und
(i) einem Oberflächenantigen auf D.eckerti, das auf SDS-PAGE-Gel unter reduzierenden Bedingungen bei im Wesentlichen 30kDa wandert,
und Beurteilen der Anwesenheit oder Abwesenheit eines Antikörper/Antigen-Komplexes in der Probe, wobei die Anwesenheit des Komplexes auf eine Nematoden-Resistenz bei dem Huftier schließen lässt, und wobei die Schleimprobe ausgewählt wird aus einer Probe aus der Nase, dem Mund, dem Rachen, dem Rektalraum oder der Vagina.

20. Verfahren zum Diagnostizieren einer Nematodeninfektion wie in Anspruch 18 beansprucht, oder Verfahren zur Bestimmung einer Nematoden-Resistenz wie in Anspruch 19 beansprucht, wobei die Schleimprobe Speichel ist.

## Revendications

1. Procédé de sélection d'ongulés qui sont génétiquement résistants aux infections par un ou plusieurs vers nématodes, ledit procédé comprenant les étapes suivantes :
(a) obtenir un échantillon de mucus dudit ongulé ;
(b) tester l'échantillon pour vérifier la présence d'un anticorps contre l'antigène de revêtement protecteur de carbohydrate (CarLA) présent au troisième stade larvaire (L3) dudit un ou plusieurs vers nématodes ; et
(c) séparer et sélectionner des ongulés dont le test d'anticorps de l'étape (b) est positif,
dans lequel l'échantillon de mucus comprend du fluide muqueux provenant du nez, de la bouche, de la gorge, de la cavité rectale ou du vagin.

2. Procédé selon la revendication 1, dans lequel l'échantillon de mucus comprend la salive.

3. Procédé selon la revendication 1, dans lequel le ou les vers nématodes sont choisis dans le groupe constitué de *Trichostrongylus colubriformis, Haemonchus contortus, Ostertagia (Teladorsagia) circumcincta, Cooperia curtical, Nematodirus spathiger, Trichostrongylus axei, Trichostrongylus vitrinus, Ostertagia ostertagi, Cooperia oncophera, Nematodirus brasilensis et Dictyocaulus eckerti.*

4. Procédé selon l'une quelconque des revendications 1 à 3, dans lequel l'anticorps comprend un anticorps monoclonal mAb PAB-1 (n° d'accès ATCC N°PTA-4005) qui se lie spécifiquement à l'antigène de surface CarLA sur le L3 du nématode.

5. Procédé pour détecter la présence d'anticorps qui sont spécifiques pour un antigène de surface L3 CarLA présent dans une ou plusieurs espèces de vers de nématode, dans un échantillon de mucus provenant d'un ongulé, ledit procédé comprenant les étapes suivantes :
(a) obtenir un échantillon de mucus dudit ongulé, comprenant du fluide muqueux provenant du nez, de la bouche, de la gorge, de la cavité rectale ou du vagin,
(b) mettre en contact ledit échantillon avec l'antigène de surface L3 CarLA, en formant ainsi un complexe anticorps/antigène ; et
(c) détecter la présence ou l'absence du complexe ;
dans lequel la présence dudit complexe anticorps/antigène est indicative d'un ongulé qui est génétiquement résistant à l'infection par le ver du nématode.

6. Procédé selon la revendication 5, dans lequel l'échantillon muqueux est la salive.

7. Procédé selon la revendication 5 ou 6, dans lequel l'antigène est choisi parmi un ou plusieurs antigènes L3 CarLA de nématodes, choisis dans le groupe constitué de :
(a) un antigène de surface sur le *C*. *curtical* qui migre sensiblement à 46 kDa et à sensiblement 22 kDa sur gel SDS PAGE dans des conditions réductrices ;
(b) un antigène de surface sur le *N.spathiger* qui migre sensiblement à 22 kDa sur un gel SDS PAGE dans des conditions réductrices ;
(c) un antigène de surface sur le *H. contortus* qui migre sensiblement à 35 kDa sur un gel SDS PAGE dans des conditions réductrices ; et
(d) un antigène de surface sur le *O.circumcincta* qui migre sensiblement à 35 à 39 kDa sur un gel SDS PAGE dans des conditions réductrices ;
(e) un antigène de surface sur *T. axei* ou *T. vitrinus* qui migre sensiblement à 35 kDa sur un gel SDS PAGE dans des conditions réductrices ;
(f) un antigène de surface sur *O. ostertagi* qui migre sensiblement à 30 à 45 kDa sur un gel SDS PAGE dans des conditions réductrices ;
(g) un antigène de surface sur *O.oncophere* qui migre sensiblement à 20 kDa et à sensiblement 45 kDa sur un gel SDS PAGE dans des conditions réductrices ;
(h) un antigène de surface sur *N. brasiliensis* qui migre sensiblement à 9 kDa et à sensiblement 12 kDa sur un gel SDS PAGE dans des conditions réductrices ; et
(i) un antigène de surface sur *D. eckerti* qui migre sensiblement à 30 kDa sur un gel SDS PAGE dans des conditions réductrices.

8. Procédé selon la revendication 7, dans lequel l'antigène est immobilisé sur une surface solide et le complexe détecté, en utilisant un marqueur sur l'antigène qui est détectable quand le complexe est formé.

9. Procédé selon la revendication 8, dans lequel le complexe est détecté en utilisant un second anticorps marqué qui se lie au complexe.

10. Procédé selon la revendication 8 ou 9, dans lequel la détection est réalisée en utilisant une bande de test comprenant un ou plusieurs antigènes immobilisés.

11. Kit permettant de détecter la présence d'un anticorps spécifique pour un ou plusieurs antigènes de surface L3 CarLA de nématodes, ledit kit comprenant :
(a) un récipient pour un échantillon de mucus ;
(b) un réactif comprenant un ou plusieurs antigènes L3 CarLA de nématodes marqués et
(c) des moyens de collecte de l'échantillon de mucus ;
dans lequel l'échantillon de mucus est choisi parmi un échantillon provenant du nez, de la bouche, de la gorge, de la cavité rectale ou du vagin.

12. Kit selon la revendication 11, comprenant :
(d) une bande de test comprenant un ou plusieurs antigènes L3 CarLA de nématode immobilisés ; et
(e) un réactif comprenant un second anticorps marqué.

13. Kit selon la revendication 11 ou 12, dans lequel le récipient d'échantillon est destiné à la salive.

14. Kit selon la revendication 11 ou 12, dans lequel les moyens de collecte sont destinés à collecter de la salive.

15. Kit selon la revendication 11, comprenant en outre un mode d'emploi.

16. Kit selon l'une quelconque des revendications 11 à 15, comprenant en outre des moyens de détection pour détecter un ou plusieurs antigènes marqués.

17. Kit selon la revendication 12, comprenant en outre des moyens de détection pour détecter un second anticorps marqué.

18. Procédé de diagnostic d'une infection par nématode chez un animal ongulé comprenant les étapes suivantes :
(a) obtenir un échantillon de mucus de l'ongulé ; et
(b) analyser l'échantillon pour vérifier la présence d'un anticorps contre un ou plusieurs antigènes L3 CarLA de nématode ;
où l'échantillon de mucus est choisi parmi un échantillon provenant du nez, de la bouche, de la gorge, de la cavité rectale ou du vagin.

19. Procédé de détermination de la résistance aux nématodes chez un ongulé, en analysant un échantillon de mucus provenant de l'ongulé pour vérifier la présence d'un anticorps spécifique pour un ou plusieurs antigènes L3 CarLA, comprenant la mise en contact de l'échantillon avec un ou plusieurs antigènes choisis dans le groupe constitué de :
(a) un antigène de surface sur le *C. curtical* qui migre sensiblement à 46 kDa et à sensiblement 22 kDa sur gel SDS PAGE dans des conditions réductrices ;
(b) un antigène de surface sur le *N. spathiger* qui migre sensiblement à 22 kDa sur un gel SDS PAGE dans des conditions réductrices ;
(c) un antigène de surface sur le *H. contortus* qui migre sensiblement à 35 kDa sur un gel SDS PAGE dans des conditions réductrices ; et
(d) un antigène de surface sur le *O.circumcincta* qui migre sensiblement à 35 à 39 kDa sur un gel SDS PAGE dans des conditions réductrices ;
(e) un antigène de surface sur *T. axei* ou T. vitrinus qui migre sensiblement à 35 kDa sur un gel SDS PAGE dans des conditions réductrices ;
(f) un antigène de surface sur *O. ostertagi* qui migre sensiblement à 30 à 45 kDa sur un gel SDS PAGE dans des conditions réductrices ;
(g) un antigène de surface sur *O.oncophere* qui migre sensiblement à 20 kDa et à sensiblement 45 kDa sur un gel SDS PAGE dans des conditions réductrices ;
(h) un antigène de surface sur *N. brasiliensis* qui migre sensiblement à 9 kDa et à sensiblement 12 kDa sur un gel SDS PAGE dans des conditions réductrices ; et
(i) un antigène de surface sur *D.eckerti* qui migre sensiblement à 30 kDa sur un gel SDS PAGE dans des conditions réductrices,
et la mesure de la présence ou de l'absence du complexe anticorps/antigène dans l'échantillon, où la présence du complexe est indicative de la résistance aux nématodes de l'ongulé, et dans lequel l'échantillon de mucus est choisi dans un échantillon provenant du nez, de la bouche, de la gorge, de la cavité rectale ou du vagin.

20. Procédé de diagnostic d'une infection par nématode selon la revendication 18, ou procédé de détermination de la résistance aux nématodes selon la revendication 19, dans lequel l'échantillon de mucus est la salive.
